# FASCICULE DE BREVET EUROPEEN

(11) **EP 1 443 872 B1**
(45) Date de publication et mention de la délivrance du brevet: **29.11.2006**
(21) Numéro de dépôt: 02793214.4
(22) Date de dépôt: 07.11.2002
(51) Int. Cl.: A61F 2/00

(54) **DISPOSITIF D'EMBALLAGE ET DE PLIAGE D'UNE PIECE EN MATERIAU SOUPLE, NOTAMMENT D'UN RENFORT PARIETAL**
VORRICHTUNG ZUM PACKEN UND FALTEN EINES STÜCKES AUS WEICHEM MATERIAL, INSBESONDERE EINES VERSTÄRKUNGSIMPLANTATS FÜR ZELLWANDEFIZITE
DEVICE FOR PACKAGING AND FOLDING A FLEXIBLE MATERIAL PART, IN PARTICULAR A PARIETAL REINFORCEMENT

(30) Priorité: 15.11.2001 FR 0114804
(43) Date de publication de la demande: 11.08.2004
(73) Titulaire: SOFRADIM PRODUCTION, 01600 Trévoux (FR)
(72) Inventeur: DETRUIT, Bernard, F-92210 Saint-Cloud (FR); THERIN, Michel, F-69004 Lyon (FR); BAILLY, Pierre, F-69300 Caluire (FR)
(74) Mandataire: Brédeville, Odile Marie
(86) Numéro de dépôt international: PCT/FR2002/003826
(87) Numéro de publication internationale: WO 2003/041614

(56) Documents cités:
- EP-A- 0 133 393
- EP-A- 0 625 334
- WO-A-95/32687
- FR-A- 2 771 622
- US-A- 4 586 930
- US-A- 5 972 008

## Description

La présente invention concerne un dispositif d'emballage et de pliage d'une pièce en matériau souple, notamment un renfort pariétal.

Certaines pièces en matériau souple doivent être stockées à plat dans leurs emballages protecteurs, afin d'éviter toute altération de leur forme plane suite à un pliage ou à un enroulement prolongé, en particulier la formation de plis, puis doivent, au moment de leur utilisation, être repliées longitudinalement ou enroulées de manière à présenter une forme ramassée en section transversale.

Tel est le cas, en particulier, d'un renfort pariétal destiné à être mis en place au moyen d'une technique de coelioscopie. Le renfort doit, en effet, être disposé à plat dans son emballage, à défaut de quoi des plis irréversibles pourraient être formés dans son matériau, empêchant ensuite de placer le renfort intimement en contact avec la paroi à traiter, en vue d'une colonisation cellulaire. Au moment de son implantation, le renfort doit être replié pour pouvoir être inséré dans un trocart afin d'être introduit dans le corps du patient par un orifice de taille réduite.

Le document EP-A-0 625 334 décrit un dispositif d'emballage et de pliage d'un renfort pariétal selon le préambule de la revendication 1.

Selon une technique existante, le renfort est simplement placé dans une boîte parallélépipédique de dimensions appropriées. Cette boîte est ouverte au moment de la mise en place du renfort, et le praticien réalise manuellement le repliage de ce renfort, avant d'insérer ce dernier dans un trocart préalablement mis en place.

Cette technique a pour inconvénient d'impliquer une manipulation du renfort, et donc, d'induire un risque de souillure de ce dernier par les gants du praticien. De plus, le renfort vient frotter contre la paroi du trocart lors de son insertion dans ce dernier, ce qui induit également un risque de contamination du renfort si la paroi du trocart est souillée.

Le brevet français n° 94 12700 propose d'insérer un fil de traction au travers du renfort, selon un parcours en créneau ou ondulé. Des tractions sur les extrémités du fil permettent de réaliser un pliage du renfort "en accordéon".

Cette technique a pour avantage d'éviter une manipulation directe du renfort pour réaliser le pliage, d'assurer un encombrement minimum du renfort avant introduction dans le trocart, et d'obtenir un pliage en accordéon, qui permet de déployer le renfort au fur et à mesure de son extraction du trocart. Par contre, cette technique laisse subsister des manipulations du renfort au moment de la saisie dans sa boîte d'emballage, et de son introduction dans le trocart. De plus, cette technique ne remédie pas au risque de souillure du renfort par frottement contre la paroi du trocart.

Une autre technique consiste également à équiper la boîte d'emballage du renfort d'une clé d'enroulement reliée à l'un des bords du renfort, et d'un tube fendu longitudinalement, dans lequel la clé d'enroulement est placée. Au moment de la mise en place du renfort, la clé est actionnée en rotation, ce qui permet de réaliser l'enroulement du renfort, et l'engagement de ce dernier dans le tube fendu. Ce tube fendu est utilisé pour introduire le renfort dans le trocart.

Cette technique a pour avantage d'éliminer toute manipulation du renfort lors de la réalisation du pliage, et de protéger le renfort contre toute souillure par frottement contre la paroi du trocart. Par contre, la fente du tube a pour inconvénient de rendre le trocart non étanche au gaz, lorsque ce tube est engagé au travers de la valve qui équipe l'extrémité proximale du trocart. Il en résulte que, lors de l'insertion de ce tube dans le trocart, il se produit une fuite du gaz carbonique utilisé pour soulever la paroi abdominale du patient afin de libérer la zone d'implantation du renfort. Ce dernier doit donc être introduit "en aveugle", ce qui est une contrainte certaine. De plus, le tube fendu présente un diamètre extérieur relativement important, de 12 mm, résultant de l'utilisation de la clé d'enroulement. Ce fort diamètre implique l'utilisation d'un trocart de diamètre interne correspondant, pas toujours adapté aux conditions d'intervention. En outre, le renfort est enroulé sur lui-même, de sorte qu'il faut le dégager entièrement du trocart pour pouvoir le déployer au niveau du site d'implantation ; ce dégagement est une contrainte notable par rapport à un pliage en accordéon tel que précité, qui permet de déployer le renfort au fur et à mesure de son extraction du trocart.

La présente invention vise à remédier précisément à ces inconvénients.

Le dispositif concerné comprend, de manière connue en soi :
- une boîte d'emballage de la pièce en matériau souple, dimensionnée pour contenir cette pièce à plat,
- des moyens de pliage permettant de réaliser le pliage de cette pièce sans manipulation directe, et
- un tube de réception, destiné à recevoir la pièce en matériau souple à l'état plié.

Selon l'invention,
- le tube de réception n'est pas fendu longitudinalement, et
- les moyens de pliage comprennent :
   - un fil de traction relié à la pièce en matériau souple, qui traverse le tube, et
   - des parois formant un entonnoir dont le fond débouche à proximité de l'ouverture du tube, par laquelle la pièce en matériau souple est destinée à être introduite dans ce tube ; cet entonnoir étant propre, lorsqu'une traction est exercée sur le fil de traction, à rabattre progressivement les zones de la pièce situées latéralement par rapport au tube vers la zone de la pièce située en regard de l'ouverture de ce tube, pour permettre l'introduction de la pièce dans le tube.

L'engagement de la pièce en matériau souple dans le tube est ainsi réalisé par simple traction sur ledit fil de traction, donc sans aucun contact direct ou manipulation de cette pièce. Les parois formant entonnoir provoquent un repliage des zones de la pièce situées latéralement par rapport au tube vers la zone de la pièce située en regard de l'ouverture de ce tube, et permettent donc d'obtenir un repliage plus ou moins "en accordéon" de cette pièce dans ce tube.

Le dispositif d'emballage et de pliage peut être réalisé avec des matériaux courants pour un emballage, à un prix de revient très réduit. Il peut notamment être réalisé par assemblage de pièces en matière synthétique thermoformées.

La pièce en matériau souple, une fois insérée dans le tube, est parfaitement protégée des souillures lors de son introduction dans un trocart. Ce tube forme de plus un moyen particulièrement commode pour réaliser cette introduction dans les meilleures conditions. Le tube peut avoir un diamètre relativement réduit, notamment de huit millimètres, qui permet son utilisation avec des trocarts de diamètres réduits correspondants ; il évite ainsi le recours à un trocart particulier pour réaliser ladite introduction, ce qui simplifie notablement l'intervention. En outre, n'étant pas fendu, le tube est étanche au gaz et ne provoque aucune fuite de gaz carbonique lors de son introduction au travers de la valve qui équipe le trocart.

Ce tube peut d'ailleurs être lui-même équipé d'une valve proximale d'étanchéité au gaz, et peut donc lui-même servir de trocart. Cette possibilité, outre la simplification qu'elle apporte, contribue à réduire le coût de l'intervention.

Avantageusement, lesdites parois formant entonnoir comprennent deux parois arrondies définissant entre elles un interstice de passage de la pièce en matériau souple, ayant une largeur inférieure à celle de cette ouverture du tube.

Ces parois arrondies s'avèrent permettre un parfait repliage de la pièce en matériau souple. Elles peuvent notamment être constituées par les parois latérales de deux plots cylindriques reliés à la boîte d'emballage.

La pièce en matériau souple pourrait, dans cette boîte, s'étendre dans le prolongement horizontal du tube, ce qui aurait toutefois pour inconvénient de conférer des dimensions importantes au dispositif et d'impliquer un surcroît de matériau. Pour cette raison, selon une forme de réalisation préférée de l'invention, le dispositif comprend un plateau qui délimite, avec la boîte d'emballage, un logement inférieur de réception de la pièce en matériau souple, qui comprend lesdites parois formant entonnoir sur sa face opposée à celle délimitant ce logement, et qui présente un bord latéral arrondi, s'étendant en retrait de la boîte, autour duquel la pièce en matériau souple est destinée à glisser lorsque la traction précitée est exercée sur le fil de traction.

Ce plateau peut comprendre des moyens de calage permettant d'immobiliser le tube de réception jusqu'à engagement complet de la pièce en matériau souple dans ce tube.

Avantageusement, le dispositif comprend en outre un couvercle conformé de manière à enserrer étroitement le tube de réception entre lui et le plateau précité.

Ce couvercle permet, conjointement au plateau, d'immobiliser parfaitement le tube de réception le temps de l'opération d'insertion, de la pièce en matériau souple dans ce tube.

Ce couvercle peut être séparable dudit plateau pour permettre l'extraction du tube de réception. De préférence, il comprend un volet pivotant permettant d'accéder au tube de réception en vue de cette même extraction.

Avantageusement, ledit plateau ou ledit couvercle, ou les deux, peut comprendre une zone en forme de "V" inversé, dont le fond est situé de manière sensiblement coaxiale à l'axe dudit tube de réception, cette zone étant destinée à être traversée par le fil de traction.

Cette zone permet ainsi de maintenir la partie du fil de traction engagé dans le tube de réception de manière parfaitement coaxiale à ce tube quel que soit l'angle selon lequel la traction est opérée sur ce fil.

Pour sa bonne compréhension, l'invention est à nouveau décrite ci-dessous en référence au dessin schématique annexé représentant, à titre d'exemple non limitatif, une forme de réalisation préférée du dispositif qu'elle concerne.
La figure 1 est une vue en perspective éclatée des pièces qui le constituent, avant conformation de l'une de ces pièces en vue de son assemblage ;
la figure 2 est une vue similaire à la figure 1 de ces mêmes pièces, après ladite conformation ;
la figure 3 est une vue de côté du dispositif, en coupe longitudinale ;
la figure 4 en est une vue en coupe selon la ligne IV-IV de la figure 6;
la figure 5 en est une vue de dessus, en cours de pliage d'un renfort pariétal qu'il contient, et
la figure 6 est une vue similaire à la figure 5, après complet pliage de ce renfort pariétal.

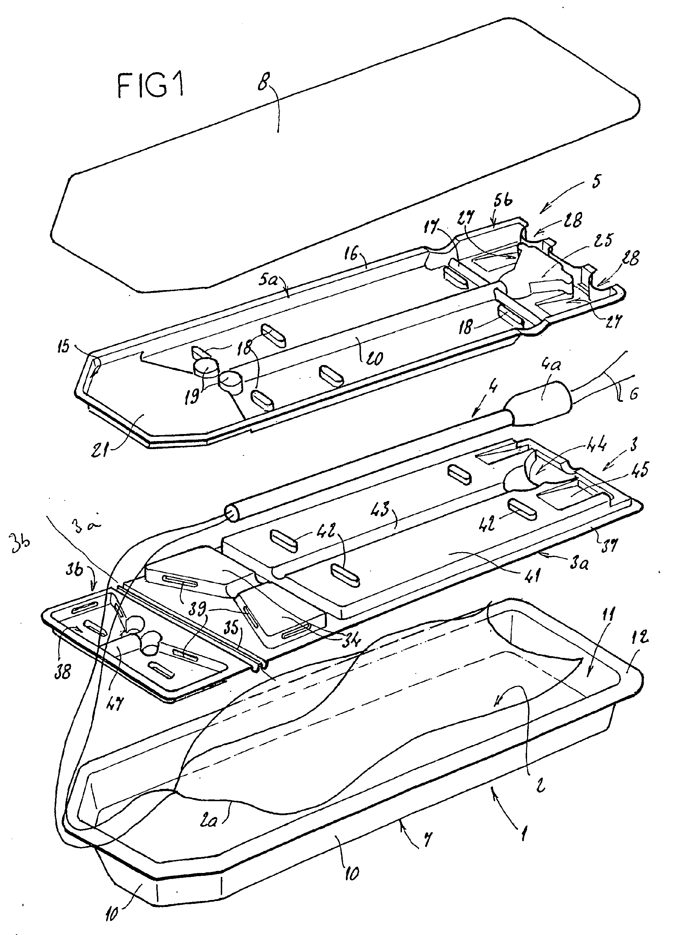

Les figures 1 à 4 représentent un dispositif 1 d'emballage et de pliage d'un renfort pariétal 2, ce renfort 2 étant destiné à être mis en place au moyen d'une technique de coelioscopie.

Lé renfort 2 est de type classique, notamment réalisé sous forme d'un tissu ou d'un tricot de fibres synthétiques, à structure ajourée. Dans l'exemple représenté sur le dessin, il a une forme générale sensiblement rectangulaire, de sorte qu'il définit un bord latéral d'extrémité 2a présentant une zone médiane.

Le dispositif 1 comprend une boîte d'emballage du renfort 2, un plateau 3, un tube 4 de réception du renfort 2 à l'état plié, un couvercle 5 et un fil de traction 6. Une boîte d'emballage supplémentaire (non représentée), recevant l'ensemble du dispositif 1, est prévue pour assurer la parfaite stérilité du dispositif.

La boîte d'emballage du renfort 2 est formée par un fond 7 et un opercule 8.

Le fond 7 comprend des parois latérales 10 délimitant une cavité 11 et un rebord périphérique extérieur 12 situé au niveau de l'ouverture de la cavité 11.

L'opercule 8 est scellé sur le rebord 12 de manière à être pelable.

Le couvercle 5 est constitué par une pièce en matière synthétique thermoformée. Il présente des parois 15 permettant son emboîtement étroit dans l'ouverture du fond 7 et un rebord périphérique 16 destiné à venir en appui contre le rebord 12 à l'issue de cet emboîtement.

Le couvercle 5 se subdivise en une partie principale 5a et une partie marginale 5b, cette dernière étant reliée à la partie principale 5a le long d'une ligne transversale de pliage 17. Cette ligne 17 forme une charnière qui permet le repliage de la partie 5b vers le haut par rapport à la partie 5a.

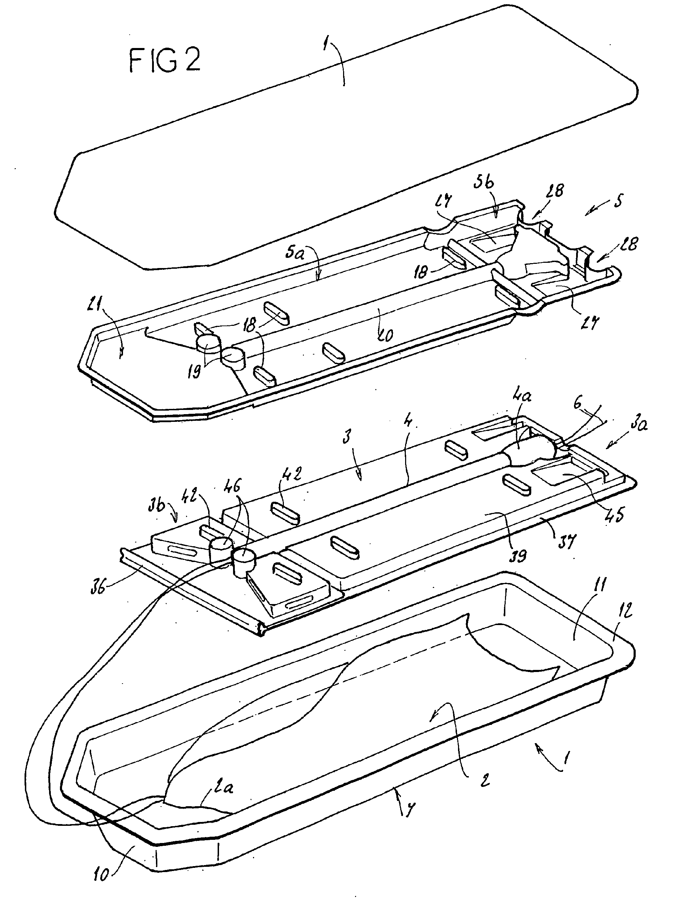

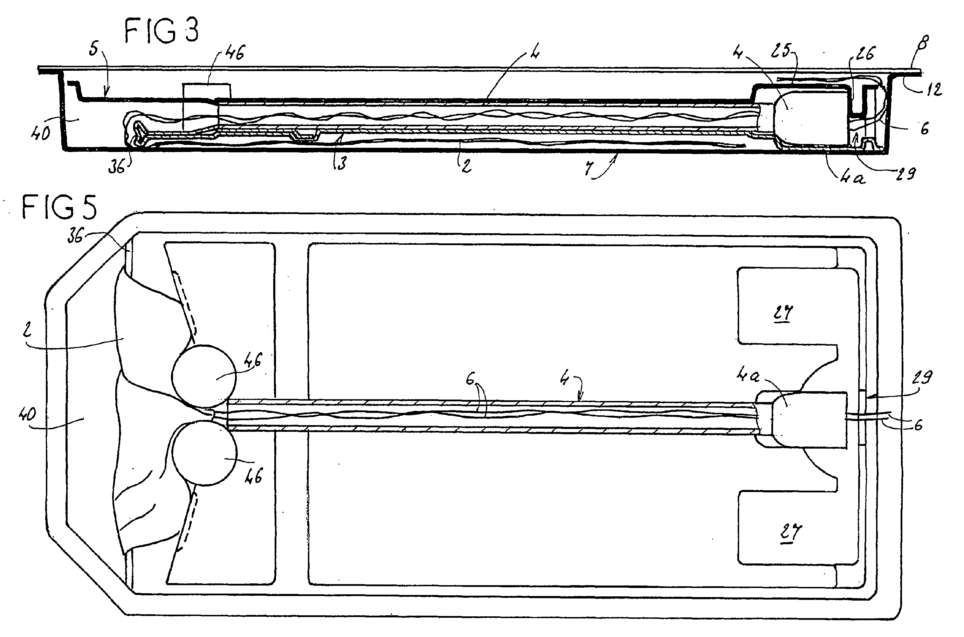

La partie 5a présente six plots oblongs 18 et deux plots cylindriques 19 délimitant, sur la face inférieure du couvercle 5, des cavités destinées à recevoir étroitement des plots respectifs correspondants 42, 46 (cf. figures 2 et 3) que comprend le plateau 3 afin de permettre l'assemblage du couvercle 5 et du plateau 3.

Cette partie 5a présente également un renfoncement allongé 20 qui délimite un logement hémicylindrique débouchant sur la face inférieure de couvercle 5, ce logement étant destiné à recevoir étroitement le tube 4 comme le montre les figures 3 à 6. Du côté opposé à la partie 5b, la partie 5a présente une paroi surélevée 21.

La partie 5b présente un bossage 25 délimitant, sur la face inférieure de couvercle 5, un logement destiné à recevoir une partie proximale élargie 4a du tube 4. Ce bossage 25 se termine, du côté opposé à la partie 5a, par une paroi 26 perpendiculaire à l'axe du logement délimité par le renfoncement 20 (cf. figure 3).

Cette partie 5b présente en outre des cavités latérales 27 et, du côté opposé à la partie 5a, deux zones arrondies qui délimitent des cavités 28. Ces cavités 27 et 28 sont propres à recevoir les doigt d'un utilisateur, ainsi que cela apparaîtra plus loin.

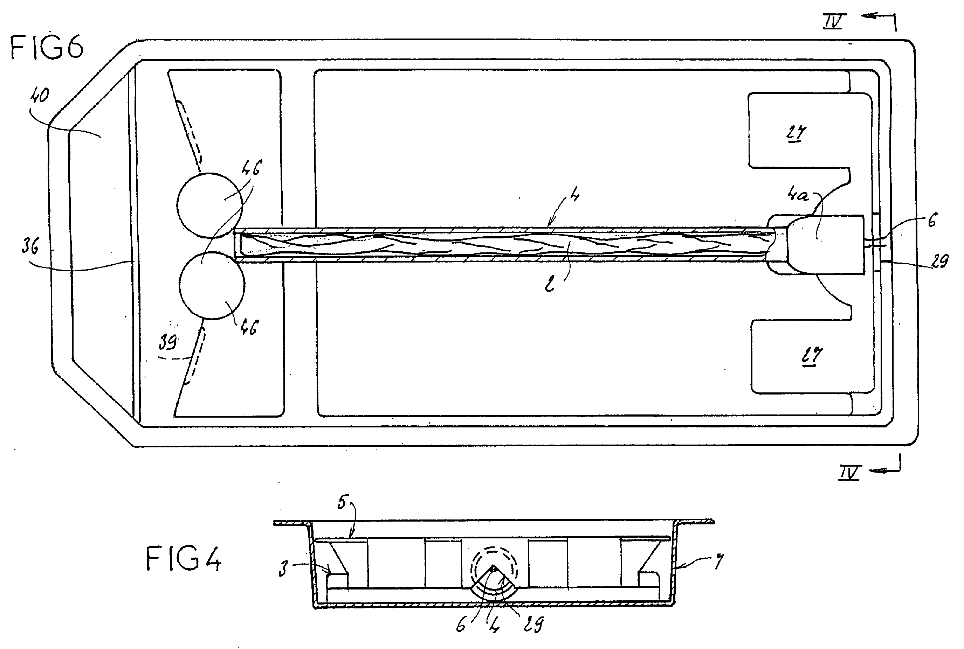

En outre, comme le montre la figure 4, la partie 5b présente, du côté opposé à la partie 5a, une encoche 29 en forme de "V" inversé, dont le fond vient sensiblement en alignement avec l'axe du logement délimité par le renfoncement 20 lorsque la partie 5b est placée dans le prolongement de la partie 5a.

Le plateau 3 est également réalisé en une matière synthétique thermoformée. Il présente une partie principale 3a et une partie marginale 3b reliées l'une à l'autre par une ligne transversale de pliage 35. Cette ligne 35 permet le repliage de la partie 3b sur la partie 3a comme cela apparaît par comparaison des figures 1 et 2. Cette ligne 35 est bordée par deux rainures à fonds arrondis, de sorte que le repliage de la partie 3b sur la partie 3a forme un bourrelet arrondi 36, relativement large.

Il apparaît sur les figures 1 et 2 que les parties 3a et 3b présentent des plots 34 et des évidements 38 correspondants, ainsi que des nervures et renfoncements 39 d'encliquetage, qui permettent l'emboîtement étroit et la rétention de la partie 3b sur la partie 3a.

La partie 3a présente un rebord périphérique 37 qui permet son positionnement dans le fond 7, avec calage. Cette partie 3a est dimensionnée de telle sorte que le bourrelet 36 se trouve en retrait de la paroi 10 correspondante du fond 7. Il existe ainsi un passage 40 entre ce bourrelet 36 et cette paroi 10 lorsque le plateau 3 est placé dans le fond 7, propre à permettre le coulissement du renfort 2 à travers lui.

La partie 3a comprend une zone centrale surélevée 41, définissant un logement inférieur de réception du renfort 2. Cette zone 41 comprend quatre plots 42, un logement hémicylindrique 43 débouchant dans la face supérieure du plateau 3, dimensionné pour recevoir étroitement le tube 4, un logement évasé 44 dimensionné pour recevoir étroitement la partie 4a du tube 4, et deux cavités 45 destinées à recevoir les parois qui délimitent les cavités 27.

La partie 3b comprend quant à elle deux plots 42 et deux plots cylindriques 46, ainsi qu'un renfoncement hémicylindrique 47 qui délimite un logement venant dans le prolongement du logement 43 lorsque la partie 3b est repliée sur la partie 3a. Ce logement est apte, avec le logement délimité par le renfoncement 20, à recevoir étroitement le tube 4 comme le montre la figure 2.

Il apparaît plus particulièrement sur les figures 5 et 6 que les deux plots 46 sont aménagés à une distance l'un de l'autre inférieure au diamètre interne du tube 4, de sorte qu'ils forment une butée contre laquelle vient se positionner l'extrémité du tube 4 lorsque ce tube 4 est placé dans les logements précités du plateau 3 et du couvercle 5. Les figures 3 et 5 montrent quant à elles que la longueur du tube 4 est telle que l'autre extrémité de ce tube 4 vient à proximité immédiate de la paroi 26. Ce tube 4 est ainsi étroitement emprisonné entre le couvercle 5 et le plateau 3 lorsque les plots 42 et la partie supérieure des plots 46 sont engagés dans les cavités délimitées par les plots 18 et 19, et est bloqué axialement lorsque la partie 5b est maintenue appliquée contre le plateau 3. Le soulèvement de cette partie 5b permet par contre d'effacer la butée que forme la paroi 26, ce qui permet le retrait du tube 4 d'entre le couvercle 5 et le plateau 3, par coulissement.

La partie 4a du tube 4 loge un clapet étanche au gaz, de type classique. Ce clapet est propre à résister à la pression du gaz carbonique utilisé pour soulever la paroi abdominale du patient pendant l'intervention, ce soulèvement permettant de libérer l'accès au site d'implantation du renfort 2.

Le fil 6 comprend deux brins formant une boucle. Il est relié à la zone médiane du bord latéral 2a précité du renfort 2 et traverse le tube 4. Sa longueur est telle qu'il peut être saisi pour exercer une traction à même de faire pénétrer le renfort 2 dans le tube 4 comme décrit ci-après.

En pratique, l'utilisateur saisit le fil 6 par une main et exerce une pression sur la partie 5b avec son autre main, en engageant son pouce et son index de cette autre main dans les cavités 27.

Une traction est exercée sur le fil 6, ce qui amène le renfort 2 à passer autour du bourrelet 36, puis à venir en contact avec les parois latérales des plots 46. Ce contact réalise, ainsi que le montre la figure 5, un rabat progressif des zones latérales du renfort 2 vers la zone médiane de ce renfort, et donc un pliage plus ou moins en accordéon du renfort 2.

La poursuite de cette traction réalise l'introduction complète du renfort 2 dans le tube 4.

Au cours de cette traction, le fil 6 vient porter contre le fond de l'encoche 29, ce qui assure le maintien de la partie du fil 6 située dans le tube 4 dans une position sensiblement coaxiale à ce tube 4, quel que soit l'angle selon lequel l'utilisateur opère ladite traction.

Une fois le renfort 2 complètement engagé dans le tube 4, les cavités 28 sont mises à profit pour soulever la partie 5b du couvercle 5 et libérer ainsi le tube 4, lequel peut être retiré d'entre le couvercle 5 et le plateau 3 par coulissement.

Le tube 4 peut ensuite être utilisé comme un trocart pour introduire le renfort 2 dans le corps du patient.

Ainsi qu'il apparaît de ce qui précède, l'invention fournit un dispositif d'emballage et de pliage d'une pièce en matériau souple, notamment un renfort pariétal 2, qui présente les avantages déterminants suivants :
- pliage du renfort 2 "en accordéon", sans aucun contact manuel avec le renfort et selon un geste simple ;
- insertion du renfort 2 dans un tube 4 de relativement faible diamètre, permettant la protection du renfort lors de l'introduction de ce renfort au travers d'un trocart ;
- étanchéité du tube 4 au gaz et possibilité d'utilisation de ce tube comme un trocart ;
- possibilité de réaliser le dispositif avec des matériaux courants pour un emballage, à un prix de revient très réduit.

Il va de soi que l'invention n'est pas limitée à la forme de réalisation décrite ci-dessus à titre d'exemple, mais qu'elle en embrasse au contraire toutes les variantes de réalisation entrant dans le champ de protection défini par les revendications ci-annexées.

## Revendications

1. Dispositif d'emballage et de pliage d'une pièce en matériau souple (2), notamment d'un renfort pariétal, comprenant :
- une boîte d'emballage de la pièce en matériau souple (2), dimensionnée pour contenir cette pièce (2) à plat,
- des moyens de pliage (6, 46) permettant de réaliser le pliage de cette pièce (2) sans manipulation directe, et
- un tube de réception (4), destiné à recevoir la pièce en matériau souple (2) à l'état plié ;
dispositif (1) **caractérisé en ce que** :
- le tube de réception (4) n'est pas fendu longitudinalement, et
- les moyens de pliage comprennent :
. un fil de traction (6) relié à la pièce en matériau souple (2), qui traverse le tube (4), et
. des parois (46) formant un entonnoir dont le fond débouche à proximité de l'ouverture du tube (4) par laquelle la pièce en matériau souple (2) est destinée à être introduite dans ce tube (4), cet entonnoir étant propre, lorsqu'une traction est exercée sur le fil de traction (6), à rabattre progressivement les zones de la pièce (2) situées latéralement par rapport au tube (4) vers la zone de la pièce (2) située en regard de l'ouverture de ce tube (4), pour permettre l'introduction de la pièce (2) dans le tube (4).

2. Dispositif selon la revendication 1, **caractérisé en ce que** le tube de réception (4) est équipé d'une valve proximale d'étanchéité au gaz.

3. Dispositif selon la revendication 1 ou la revendication 2, **caractérisé en ce que** lesdites parois formant entonnoir comprennent deux parois arrondies (46) définissant entre elles un interstice de passage de la pièce en matériau souple (2), ayant une largeur inférieure à celle de cette ouverture du tube de réception (4).

4. Dispositif selon la revendication 3, **caractérisé en ce que** les parois arrondies sont constituées par les parois latérales de deux plots cylindriques (46) reliés à la boîte d'emballage.

5. Dispositif selon l'une des revendications 1 à 4, **caractérisé en ce qu'**il comprend un plateau (3) qui délimite, avec la boîte d'emballage, un logement inférieur de réception de la pièce en matériau souple (2), qui comprend lesdites parois (46) formant entonnoir sur sa face opposée à celle délimitant ce logement, et qui présente un bord latéral arrondi (36), s'étendant en retrait de la boîte, autour duquel la pièce en matériau souple (2) est destinée à glisser lorsque la traction précitée est exercée sur le fil de traction (6).

6. Dispositif selon la revendication 5, **caractérisé en ce que** le plateau (3) comprend des moyens de calage (46, 26) permettant d'immobiliser le tube de réception (4) jusqu'à engagement complet de la pièce en matériau souple (2) dans ce tube.

7. Dispositif selon la revendication 5 ou la revendication 6, **caractérisé en ce qu'**il comprend un couvercle (5) conformé de manière à enserrer étroitement le tube de réception (4) entre lui et le plateau (3).

8. Dispositif selon la revendication 7, **caractérisé en ce que** le couvercle (5) comprend un volet pivotant (5b) permettant d'accéder au tube de réception (4) pour l'extraction de ce tube.

9. Dispositif selon l'une des revendications 5 à 8, **caractérisé en ce que** le plateau (3) ou le couvercle (5), ou les deux, comprend une zone (29) en forme de "V" inversé, dont le fond est situé de manière sensiblement coaxiale à l'axe du tube de réception (4), cette zone (29) étant destinée à être traversée par le fil de traction (6).

## Claims

1. A device for packaging and folding a flexible material part (2), especially a parietal reinforcement, comprising:
- a packaging box for the flexible material part (2), dimensioned so as to hold this part (2) flat,
- folding means (6, 46) allowing this part (2) to be folded without direct handling, and
- a receiving tube (4), designed to receive the flexible material part (2) in the folded state,
the device (1) being **characterized in that**:
- the receiving tube (4) is not longitudinally slit, and
- the folding means comprise:
• a traction suture (6) connected to the flexible material part (2), which passes through the tube (4), and
• walls (46) forming a funnel, the base of which emerges close Lo the opening an the tube (4) through which the flexible material part (2) is designed to be introduced into this tube (4), this funnel being fit, when a traction is applied to the traction suture (6), to progressively fold down those zones of the part (2) which are situated laterally relative to the tube (4) toward that zone of the part (2) which is situated opposite the opening in this tube (4), to allow the part. (2) admission to the tube (4).

2. The device as claimed in claim 1, **characterized in that**, the receiving Lube (4) is equipped with a proximal gas-tight valve.

3. The device as claimed in claim 1 or claim 2, **characterized in that** said funnel-forming walls comprise two rounded walls (46) defining between them a pass-through gap for the flexible material part (2) having a width less than that of this opening in the tube (4).

4. The device as claimed in claim 3, **characterized in that** the rounded walls are constituted by side walls of two cylindrical blocks (46) connected to the packaging box.

5. The device as claimed in one of claims 1 to 4, **characterized in that** it comprises a tray (3) which delimits, with the packaging box, a bottom receptacle for receiving the flexible material part (2), comprises said funnel-forming walls (46) on its face opposite that delimiting this receptacle, and has a rounded side edge (36), extending back from the box, around which the flexible material part (2) is designed to slide when the aforesaid traction is applied to the traction suture (6).

6. The device as claimed in cLaim 5, **characterized in that** the tray (3) comprises wedging means (46, 26) allowing the receiving tube (4) to be immobilized until the flexible material part (2) is fully engaged in this tube.

7. The device as claimed in claim 5 or claim 6, **characterized in that** it comprises a cover (5) configured such as to grip the receiving tube (4) tightly between it and the tray (3).

8. The device as claimed in claim 7, **characterized in that** the cover (5) comprises a hinged flap (5b), allowing access to the receiving tube (4) for the extraction of this tube.

9. The device as claimed in one of claims 5 to 8, **characterized in that** the tray (3) or the cover (5), or both, comprises a zone (29) in the shape of an inverted "V", the base of which is situated substantially coaxially to the axis of the receiving tube (4), this zone (29) being designed to be passed through by the traction suture (6).

## Patentansprüche

1. Vorrichtung zum Verpacken und Zusammenfalten eines Stückes at s flexiblem Material (2), insbesondere einer parietalen Verstärkung, mit:
- einer Schachtel zum Verpacken des Stückes aus flexiblem Material (2), d e so bemessen ist, dass sie dieses Stück (2) im flachen Zustand beinhalten kann,
- Mitteln zum Zusammenfalten (6, 46), die das Zusammenfalten des Stückes (2) ohne unmittelbare Manipulation ermöglichen, und
- einem Aufnahmerohr (4), das dazu bestimmt ist, das Stück aus flexiblern Material (2) im zusammengefalteten Zustand aufzunehmen;
wobei die Vorrichtung (1) **dadurch gekennzeichnet ist, dass**:
- das Aufnahmerohr (4) nicht in Längsrichtung geschlitzt ist, und
- die Mittel zum Zusammenfalten aufweisen:
. einen Zugfaden (6), der mit dem Stück aus flexiblem Material (2) verbunden ist, der durch das Rohr (4) hindurchgeht, und
. Wände (46), die einen Trichter bilden, dessen Boden nahe der Öffnung des Rohrs (4) mündet, durch die das Stück aus flexiblem Material (2) in das Rohr (4) eingeführt werden soll, wobei, wenn auf den Zugfaden (6) ein Zug ausgeübt wird, der Trichter geeignet ist, die bezüglich des Rohrs (4) seitlich befindlichen Bereiche des Stückes (2) nach und nach zu dem der Öffnung des Rohrs (4) gegenüberliegend befindlichen Bereich des Stückes (2) umzuschlagen, um das Einführen des Stückes (2) in das Rohr (4) zu ermöglichen.

2. Vorrichtung nach Anspruch 1, **dadurch gekennzeichnet, dass** das Aufnahmerohr (4) mit einem proximalen Ventil zur Abdichtung gegenüber Gas ausgestattet ist.

3. Vorrichtung nach Anspruch 1 oder 2, **dadurch gekennzeichnet, dass** die Wände, die den Trichter bilden, zwei abgerundete Wände (46) aufweisen, die zwischen sich einen Zwischenraum für den Durchgang des Stückes aus flexiblem Material (2) definieren, der eine Breite aufweist, die geringer ist als diejenige der Öffnung des Aufnahmerohrs (4).

4. Vorrichtung nach Anspruch 3, **dadurch gekennzeichnet, dass** die abgerundsten Wände durch Seitenwände zweier zylindrischer Zapfen (46) gebildet sind, die mit der Schachtel zum Verpacken verbunden sind.

5. Vorrichtung nach einem der Ansprüche 1 bis 4, **dadurch gekennzeichnet, dass** sie ein Tablett (3) aufweist, das mit der Schachtel zum Verpacken eine untere Kanmer zur Aufnahme des Stückes aus flexiblem Material (2) abgrenzt, und das auf seiner der Seite, die die Kammer abgrenzt, entgegengesetzien Seite die den Trichter bildenden Wände (46) aufweist, und das einen abgerundeten seitlichen Rand (36) aufweist, der sien von der Schachtel zurückversetzt erstreckt, und um den das Stück aus flexiblem Material (2) gleiten kann, wenn auf den Zugfaden (6) der vorstehend genannte Zug ausgeübt wird.

6. Vorrichtung nach Anspruch 5, **dadurch gekennzeichnet, dass** das Tablett (3) Mittel zum Festlegen (46, 26) aufweist, um das Aufnahmerohr (4) bis zum vollständigen Eingreifen des Stückes aus flexiblem Material (2) in dieses Rohr unbeweglich halten zu können.

7. Vorrichtung nach Anspruch 5 oder Anspruch 6, **dadurch gekennzeichnet, dass** sie einen Deckel (5) aufweist, der so ausgestaltet ist, dass er das Aufnahmerohr (4) zwischen sich und dem Tablett (3) fest einfasst.

8. Vorrichtung nach Anspruch 7, **dadurch gekennzeichnet, dass** der Deckel (5) einen verschwenkbaren Flügel (5b) aufweist, der es ermöglicht, zum Herausziehen des Rohrs Zugang zu dem Aufnahmerohr (4) zu verschaffen.

9. Vorrichtung nach einem der Ansprüche 5 bis 8, **dadurch gekennzeichnet, dass** das Tablett (3) oder der Deckel (5) oder beide einen Bereich (29) in Form eines umgekehrten "V" aufweist/aufweisen, dessen Boden etwa koaxial mit der Achse des Aufnahmerohrs (4) angeordnet ist, wobei der Bereich (29) dazu bestimmt ist, von dem Zugfaden (6) durchquert zu werden.
